# EUROPEAN PATENT SPECIFICATION

(11) **EP 4 522 234 B1**
(45) Date of publication and mention of the grant of the patent: **27.08.2025**
(21) Application number: 23713167.7
(22) Date of filing: 15.03.2023
(51) Int. Cl.: A61L 15/12, A61F 5/01, A61F 5/058

(54) **CURABLE IMMOBILIZING CAST DRESSING**
HÄRTBARER IMMOBILISIERENDER GUSSVERBAND
PANSEMENT COULÉ IMMOBILISANT DURCISSABLE

(30) Priority: 13.05.2022 EP 22173317
(43) Date of publication of application: 19.03.2025
(73) Proprietor: UV CAST SPOLKA Z OGRANICZONA ODPOWIEDZIALNOSCIA, 15-424 Bialystok (PL)
(72) Inventor: GRABOWSKI, Rafal, 00-232 Warszawa (PL); KROCZEK, Beata, 05-091 Zabki (PL); KUPAJ, Szymon, 05-500 Piaseczno (PL)
(74) Representative: AOMB Polska Sp. z.o.o.
(86) International application number: PCT/IB2023/052525
(87) International publication number: WO 2023/218254

(56) References cited:
- WO-A2-2010/017817
- US-A- 5 913 840

## Description

### Technical field

The invention relates to a curable immobilisation cast dressing comprising a substrate element in form of a flexible sleeve made of knitted fabric with a meshed weave, and a curable element in form of a flexible sleeve made of knitted fabric with a meshed weave, soaked in a curable material, intended to be slid onto the substrate element. Both elements are made of synthetic yarn with hydrophobic properties. The curable material constitutes a hybrid mixture of materials undergoing curing upon exposure to light and water. The invention is useful in orthopaedics, primarily for temporal immobilisation of broken or damaged limbs in order to ensure their proper healing.

### Prior Art

Immobilisation dressings are primarily intended for the immobilisation of broken limbs, in order to ensure proper conditions for a safe fusion of the damaged bones during the healing process, and therefore preventing mutual displacement of the damaged bone fragments, as well as for relieving the broken limb. Immobilisation dressings are also used to protect minor injuries, such as sprains or joint dislocations.

In the past, such dressings were made primarily of cotton bandages applied layerwise onto a damaged limb, with layers of paste made of gypsum (calcium sulphate hemihydrate) mixed with water being placed between the individual layers of the bandage, which underwent curing as a result of full hydration of calcium sulphate, creating a rigid shell, in the case of a limb usually having the form of a sleeve. The drawbacks of such traditional solutions included laborious procedure of applying the dressing, relatively long curing time and quite heavy weight of the dressing, especially in the case of larger sizes (e.g. covering an entire leg or arm with a part of a shoulder or a chest). Moreover, the solid and impermeable shell of the plaster cast dressing provides very limited access of air to skin surface located below the cured dressing and favours the accumulation of moisture, which - combined with the inability to perform normal hygienic procedures such as a shower or a bath - not only causes the patient's discomfort, but also contributes to skin irritation under the dressing, excess sweating, growth of fungi or occurrence of inflammations. A plaster cast dressing cannot be wetted, as it diminishes its strength.

Moreover, the rough inner surface of a cured plaster cast dressing can cause local mechanical damage due to rubbing or pressure.

Nowadays, materials used more often instead of plaster cast include thermoplastic or fibreglass soaked in polyurethane resin, which allow for solving at least some of the problems existing in the case of plaster cast dressings.

For example, US2008319362 (A1) discloses an immobilisation dressing arrangement comprising a substrate insulating layer, an intermediate layer of a thermoplastic polymer, such as poly(ethylene terephthalate) (PET), poly(vinyl chloride) (PVC), polycaprolactone or polyethylene, and an outer protective layer. Adjustment of the dressing form to the limb shape takes place by supplying heat to the middle layer in order to give it sufficient plasticity. Upon application onto a limb, the dressing is cooled down, which results in solidifying of the middle layer in the desired form. The substrate layer protects the patient's skin against excess heating during application and cooling of the dressing.

US6585671B2 in turn describes an immobilisation dressing consisting of a meshed sleeve woven of a flexible fibre and a second layer in form of a stocking, preferably made of polypropylene, pulled over this sleeve. Before applying onto the patient's body part, usually a limb, the sleeve is soaked in a curable material, such as a synthetic resin. The amount of the curable material and the mesh size of the sleeve are adjusted so that after the curing the dressing would comprise small openings providing ventilation. This description does not specify any particular curable material or curing method.

WO2010017817 describes an irradiation assembly for curing the material of an orthopaedic bandage upon applying a dressing immobilising the patient's limb, the assembly comprising a shell-like polymeric structure with at least one built-in source of light capable of emitting radiation within a range of 200-750 nm and a power source. The shell-like structure has the shape of a trough, or it is flexible to an extent enabling its rounding, e.g. on the elbow or knee joint. The shell-like structure is intended to cure a bandage soaked in a polymer crosslinkable upon exposure to light, in a shape substantially corresponding to the form of the inner surface of this structure.

WO2006132961 discloses an assembly comprising a nonwoven material, for example in form of a tape or sheet, soaked or covered with a mixture of substances capable of curing once exposed to an activating factor, which in particular can be moisture.

WO2016071873 discloses a curable dressing in form of a flat sheet with a meshed structure, into the openings of which a curable material is introduced. In order to immobilise a limb, the sheet is wound around the limb and fixed in the final position by means of suitable elements binding the edges of the sheet, followed by curing the curable material, such as, e.g. epoxy resin, filling the openings of the meshed structure.

The drawback of the known solutions using curable materials exposed to moisture was the excessively rapid curing, making it more difficult for a physician to arrange the damaged limb in the proper target position. Dressings curable upon exposure to water or moisture from the air provide no control over the curing time, as the cross-linking process is started immediately after unpacking the dressing. As a result such dressings lose their plasticity relatively fast, significantly limiting the time available for a physician to properly adjust the dressing shape to a limb or a part thereof to be immobilized. Further, due to exclusively water/moisture-based curing mechanism the use of such dressings is difficult to standardize as the dressing curing rate can vary depending on the actual moisture content in the air or the way the dressing is soaked in water. For these reasons, synthetic, light immobilisation dressings are usually used in the case of limbs only requiring immobilisation in the original position, while in the case of limbs requiring resetting prior to immobilisation, traditional plaster cast dressings are still in frequent use.

The use of materials curable upon exposure to light is limited to surfaces which are accessible to light. Any region of the dressing unreachable to light, such as its inner side facing the limb or its internal structure (in case of thick dressings), is highly likely to contain unreacted photopolymer remains throughout the entire period of use, this posing serious threat to the patient's health, in particular in case of allergies. This limitation requires either applying the dressing in thin layers and curing each layer separately, or using specialised tips of light sources, enabling the irradiation of less accessible areas, although the latter is only possible in a very limited range anyway.

Another drawback of known solutions was the necessity to use an oscillating saw or another similar tool in order to remove the dressing. Such a procedure requires high precision so as not to damage the limb when cutting the cured shell of the immobilisation dressing. US5913840A discloses a reinforceable orthopedic casting article, comprising a backing and a curable resin comprising an activator, wherein the activator comprises an active reducing component of a redox pair and is present in a sufficient amount to assist in the initiation of the cure of an addition polymerizable reinforcement compound, and thereby reinforce the treated area of the casting article. The present invention differs from the immobilisation cast known from D1 in that the cast dressing is composed of two flexible sleeves comprising a knitted fabric and a meshed weave, i.e. a substrate element and a curable element, wherein the curable element is suitable to be slid onto the substrate element, whereby only the curable element is soaked in a curable mixture.

### Summary of the invention

It was the aim of the present invention to solve the abovementioned problems occurring in the case of prior solutions, in particular by providing a curable immobilisation cast dressing of a small mass, smaller thickness, good breathability, full waterproofness, providing the patient with the possibility of an easy bath or shower with no negative effects both for the mechanical properties of the dressing as well as for the condition of the skin under the dressing. Moreover, it was the aim of the invention to provide a dressing, the application procedure of which would be considerably shorter and easier compared to the traditional immobilising plaster casts, at the same time ensuring very good fitting of the dressing form to the individual shape of the patient's limb, and low flexibility of an already cured dressing, leading to an improvement of the patient's comfort. Another very important aim of the invention was to ensure control over the dressing curing time, so as to allow a physician to reset the limb or to model the dressing without the rush caused by fast and uncontrollable curing of a polymer dressing removed from a package. A further aim of the invention was to facilitate the dressing removal procedure, and in particular to eliminate the necessity to use power tools such as an oscillating saw. Another aim of the invention was to facilitate the procedure of adjusting the proper size of the dressing to the patient's limb size, without the necessity to trim the dressing. These aims have been achieved due to the development of a solution constituting the subject-matter of the present invention.

The subject-matter of the invention is a curable immobilisation cast dressing comprising a substrate element in form of a flexible sleeve made of knitted fabric with a meshed weave, and a curable element in form of a flexible sleeve made of knitted fabric with a meshed weave, soaked in a curable material, intended to be slid onto the substrate element. Both elements are made of synthetic yarn with hydrophobic properties. The curable material is a hybrid mixture comprising:
(a) at least one compound undergoing polymerisation and/or crosslinking upon exposure to light, preferably with a wavelength of 405 nm, forming at least 50% by weight of the hybrid mixture;
(b) at least one photoinitiator;
(c) at least one compound undergoing polymerisation and/or crosslinking upon exposure to water;
(d) at least one catalyst of the reaction of polymerisation and/or crosslinking of the compound (c) upon exposure to water.

Preferably the compound (c) undergoing polymerisation and/or crosslinking upon exposure to water is used in an amount of at least 20% by weight of the hybrid mixture

Preferably, the compound (a) undergoing polymerisation and/or crosslinking upon exposure to light at the same time is the compound (c) undergoing polymerisation and/or crosslinking upon exposure to water.

Preferably, said at least one compound (c) undergoing polymerisation and/or crosslinking upon exposure to water comprises isocyanate groups. In this embodiment of the dressing according to the invention, a tertiary amine is preferably used as the catalyst (d), in particular bis[2-N,Ndimethylamino)ethyl]ether (CAS no. 3033-62-3) or 2,2-dimorpholinodiethylether (DMDEE; CAS no.: 6425-39-4).

Preferably, the curable element along its longitudinal axis has a cutting line marked by a thinner weave of the knitted fabric.

Preferably, the substrate element has a lateral branch in form of a sleeve with a smaller diameter, constituting a thumb guard, while the curable element has an opening for the thumb. Preferably, the dressing according to the invention has a colour marking defining its size, preferably in form of a colour thread finish. In a particularly preferable embodiment of the dressing according to the invention, the colour marking additionally indicates the location of the opening for the thumb.

### Detailed description of the invention

The curable immobilisation cast dressing according to the invention comprises a substrate element in form of a flexible sleeve made of knitted fabric with a meshed weave made of synthetic yarn with hydrophobic properties, and a curable element in form of a flexible sleeve made of knitted fabric with a meshed weave, soaked in a curable material. The substrate element can have one or several layers. Preferably, it is made of synthetic yarn, preferably polyester. The hydrophobic properties of the yarn ensure its waterproofness, which combined with the meshed weave of the knitted fabric makes it easier for the air to reach the skin under the dressing and prevents the accumulation of moisture on its surface, protecting the skin against maceration. The primary role of the substrate element is to ensure the patient's comfort by isolating the cured part of the dressing from direct contact with the skin, protect the skin against abrasion, and provide the ability to maintain proper hygiene of the skin surface.

During application of the dressing, the curable element, also in form of a flexible sleeve made of knitted fabric with a meshed weave, is slid onto the substrate element in form of a sleeve. The curable element is soaked in a curable material constituting a hybrid mixture comprising:
(a) at least one compound undergoing polymerisation and/or crosslinking upon exposure to light, preferably with a wavelength of 405 nm, forming at least 50% by weight of the hybrid mixture;
(b) at least one photoinitiator;
(c) at least one compound undergoing polymerisation and/or crosslinking upon exposure to water;
(d) at least one catalyst of the reaction of polymerisation and/or crosslinking of the compound (c) upon exposure to water.

The meshed weave of both sleeves - the substrate element and the curable element - ensures flexibility while being put onto a limb, while upon curing the curable element it enables sufficient access of air to the skin surface, prevents the accumulation of moisture and helping to maintain proper hygiene of the skin area located under the dressing.

In the context of the present invention, the term *light* is to be understood as electromagnetic radiation with a wavelength in a range of 150 - 750 nm. The preferable wavelength of the light used to cure the dressing according to the invention is 405 nm.

The compound (a) undergoing polymerisation and/or crosslinking upon exposure to light is to be understood as a compound comprising functional groups, which upon exposure to light and in the presence of a photoinitiator undergo a reaction with the formation of a spatial structure. For example, such functional groups are vinyl groups, and in particular acrylic and methacrylic groups.

The compound (c) undergoing polymerisation and/or crosslinking upon exposure to water is in turn to be understood as a compound comprising functional groups, which upon exposure to water, preferably in the presence of a catalyst, undergo a reaction with the formation of a spatial structure. For example, such functional groups are isocyanate groups.

In one of the embodiments of the dressing according to the invention, the hybrid mixture comprises the compound (a) undergoing polymerisation and/or crosslinking upon exposure to light, which at the same time is the compound (c) undergoing polymerisation and/or crosslinking upon exposure to water. This means that such a compound at the same time comprises in its structure functional groups enabling polymerisation and/or crosslinking upon exposure to light (in the presence of a photoinitiator), as well as functional groups enabling polymerisation and/or crosslinking upon exposure to water. Examples of such compounds include polyurethane prepolymers functionalised with 2-hydroxyethyl methacrylate, comprising at the same isocyanate groups, capable of reacting with water and self-crosslinking, and acrylic groups with a free double bond, capable of curing upon exposure to light.

Moreover, the hybrid mixture can comprise catalysts, stabilizers, viscosity adjusting agents, colouring agents, components facilitating processing and homogenisation as well as fillers.

The composition of start hybrid mixture has a direct impact on its performance characteristics and organoleptic features. Use of proper weight ratios of the mixture components (micromolecular to oligomeric to polymeric to the remaining compounds) directly affects the rheological properties of the hybrid mixture, and therefore the easiness of soaking the knitted fabric of the dressing, and the affinity to the material the knitted fabric is made of. Choice of the mixture composition allows for easy application of the immobilising sleeve and improves the hygiene of the personnel's work by minimising the leakage of the mixture from the knitted fabric soaked therein.

Due to the use of the components undergoing crosslinking and polymerisation upon exposure to two various factors: light and water in a single mixture, it is possible to obtain a twofold benefit.

On the one hand, due to the use of at least one component undergoing curing upon exposure to water, there is a minimised risk of only partial curing of the photocurable components, resulting, e.g. from the impossibility of efficient light penetration, that used to limit the efficiency of activating the photoinitiator molecules. The reason behind limited light transmittance can be, e.g. a mechanical barrier generated by the fibres of the dressing material. On the other hand, the use of at least one photocurable component allows for a more precise control of the curing rate of the mixture, compared to what is possible in case of systems comprising only substances undergoing curing in reaction with water, wherein the curing rate depends primarily on the composition of the initial mixture. In case of photocurable components, the reaction initiation is influenced primarily by the time of starting irradiation by means of a dedicated light source, which is much easier to choose and control by the personnel responsible for the dressing application.

The mixture of polymers, oligomers and/or monomers suitable for polymerisation upon exposure to light, along with an addition of a photoinitiator, is called a photopolymer.

Curing of the photopolymer/s contained in the hybrid mixture upon exposure to light radiation, preferably with a wavelength of 405 nm, is initiated by the disintegration of the photoinitiator/s, that is the compounds which, upon exposure to light, undergo homolytic disintegration generating reactive molecules (radicals, ion radicals, macroradicals). Such reactive molecules subsequently become active centres initiating the reactions of polymerisation and/or crosslinking occurring between the molecules contained in the photopolymer. Use of a proper photoinitiator and/or a system of photoinitiators has a direct impact on the polymerisation reaction rate, which depends on the number of active centres provided at a given time. In the following steps the reactive molecules (or macromolecules) can be capable of further propagation in reactions of polymerisation and/or crosslinking, forming a mechanically robust structure along with the knitted fabric material. Exemplary photoinitiators are phosphine derivatives, such as diphenyl(2,4,6-trimethylbenzoyl)phosphine oxide.

The component/components of the hybrid mixture is/are cured upon exposure to water due to the presence - within their chemical structure - of functional groups capable of reacting with water molecules. Examples of this type of substances include all kinds of polyurethane prepolymers and/or their copolymers, comprising free isocyanate groups. Depending on the number and distribution of the functional groups, in further steps the polymers generated in this way can react with the remaining components of the hybrid mixture to form spatial structures. In case of this curing mechanism, the reaction rate depends directly on the initial composition of the hybrid mixture, and on the amount of water supplied from the outside to the reaction area.

The double mechanism of curing the hybrid mixture enables controlling the curing time of the dressing. Upon removing the dressing from its package, a physician has enough time to calmly and carefully prepare the limb and properly put on the dressing without risking its premature curing. The decision about the moment of curing of the dressing is made by the medical personnel providing light and water, so that the physician can reset the limb or model the dressing with no hurry. **In** practice, usually after putting the substrate element sleeve on the limb, sliding the curable element sleeve thereon and modelling the dressing, the process of initial irradiation is performed first, initiating the photopolymerisation process, followed by the drying process resulting in final curing, said step following the mechanism involving water. The irradiation starting time and the photoinitiator choice allow for controlling the photopolymerisation step rate, provides the physician with sufficient time for proper preparation of the limb and the dressing prior to its curing.

**In** other terms, it is the physician who decides when he/she wants to start curing the dressing. Exposure of the dressing to UV radiation (preferably having a wavelength of 405 nm) initiates the curing process, while the addition of the prepolymer is only of auxiliary nature, allowing the dressing to be cured fully also in these regions, which cannot be reached by UV radiation, e.g. between the fibers or at the inner side of the dressing (contacting the substrate element). The prepolymers undergoing polymerisation and/or crosslinking upon exposure to water used in the dressing of the invention show prolonged curing time, so that it is the UV radiation that is the first and main mechanism responsible for curing the dressing.

Preferably, the curable element sleeve along its longitudinal axis has a cutting line defined by a thinner weave of the knitted fabric, which allows for removing the dressing without the use of power tools such as an oscillating saw, but solely by cutting the individual transverse fibres arranged on the cutting line by means of scissors. As the substrate element sleeve is made of substantially uncured knitted fabric, it does not require an analogical cutting line, because it can be cut with scissors.

In one of the preferred embodiments, when the curable dressing is intended for use on the forearm, the substrate element has a lateral branch in form of a sleeve with a smaller diameter, constituting a thumb guard, while the curable element has an opening for the thumb. Such a solution allows for positioning the hand in a natural position and for keeping the dressing on the limb more securely, and at the same time for avoiding abrasion and irritation of the thumb skin by the opening edge of the cured outer element.

In one of the preferred embodiments, the dressing according to the invention has a colour marking defining its size, preferably in form of a colour thread finish. For example, it can be the dressing size marking according to the scheme: small (S) - green, medium (M) - red, large (L) - blue. Due to such marking, when fitting the dressing or during optional replacement of the dressing, the physician's quick choice of the size is facilitated. This is relevant, e.g. in a situation when the original dressing was applied to initially heavily swollen limb, and once the swelling goes away it may be necessary to replace the dressing with a smaller one so as to avoid excess clearance between the limb and the cured layer of the dressing. In case of the embodiment of the dressing with a thumb opening, the colour marking additionally indicates the location of this opening, making it easier for the physician to quickly and properly apply the dressing. Use of a curable dressing in various sizes eliminates the necessity of trimming, and therefore it expedites the process of applying the dressing.

In the second, alternative aspect, the invention relates to a curable immobilisation cast dressing comprising a substrate element and a curable element, the substrate element having the form of a flexible sheet and the curable element having the form of a flexible splint with dimensions which allow for surrounding from 1/3 to 2/3 of the limb circumference. Both elements are made of knitted fabric with a meshed weave, made of synthetic yarn with hydrophobic properties. The curable element is soaked in a curable material in form of a hybrid mixture comprising the same components (a)-(d) which are listed above in relation to the dressing according to the first aspect of the invention. This element is intended to press the substrate element against the limb with fixing means. Exemplary fixing means can include a bandage, flexible bands, binding clips, straps with buckles as well as hook and loop type fastenings (Velcro^{®}). When applying the dressing, the physician forms the sheet of the curable element into the shape of a splint, adjusting the dimensions of the sheet so that the created splint would cover from 1/3 to 2/3 of the limb circumference.

### Examples

The orthopaedic immobilisation dressing according to the invention and the achieved effect are illustrated by the following embodiments:

### Example 1

A meshed, knitted fabric textile sleeve made of polyester-elastomer yarn [of 300DEN F96 x 2 polyester thread from raw textured white heavy strain polyester and a polyether-polyurethane copolymer (PA06 78/24/2 DTX Polyurethane (Spandex) 22 DTX Double strain textured polyamide)] was submerged in 201 g of a mixture of:
(i) 100 g of a photopolymer comprising the esterification products of 4,4'-isopropylidenediphenol, ethoxylated, and 2-methylprop-2-enoic acid, silanised dental glass, diphenyl(2,4,6-trimethylbenzoyl)phosphine oxide (VarseoSmile Temp from the BEGO Bremer Goldschlägerei Wilh. Herbst GmbH & Co. KG Company);
(ii) 100 g of methylene diphenyl diisocyanate (MDI)-based aromatic polyisocyanate prepolymer (Desmodur^{®} E 23 from the Covestro AG Company);
(iii) 1 g of bis[2-N,N-dimethylamino)ethyl]ether.

Subsequently, the excess mixture was squeezed out of the sleeve, resulting in 85% sleeve mass increase compared to a non-soaked dressing. The soaked sleeve formed in this way was protected by a sealing package.

Rectangular templates for mechanical tests with dimensions of 25 mm x 80 mm and approx. 4 mm in thickness were cut out of the formed soaked sleeve. Upon irradiating for 3 minutes with UV radiation of the wavelength of 405 nm, followed by moistening, dust-dry and rigid templates were produced. Mechanical tests of flexural strength were performed (according to the standard PN-EN ISO 178) along with resilience using Charpy's method (according to the standard PN - EN ISO 179-1). The templates were characterised by flexural strength of 1.5 MPa and resilience of 8.49 kJ/m².

### Example 2

A template was obtained in a procedure as defined in the Example 1 above, with the following differences:
- bisphenol A ethoxylate dimethacrylate (CAS no.: 41637-38-1) together with a photoinitiator = diphenyl(2,4,6-trimethylbenzoyl)phosphine oxide (TPO; CAS no.: 75980-60-8) were used as the component (i);
- 2,2-dimorpholinodiethylether (DMDEE; CAS no.: 6425-39-4) was used as the component (iii).

Subsequently, the excess mixture was squeezed out of the sleeve, resulting in 500-600% sleeve mass increase compared to a non-soaked dressing. The soaked sleeve formed in this way was protected by a sealing package.

The templates obtained in the same form and tested in the same way as in the example 1, were characterised by flexural strength of 1.69 MPa and resilience of ,27 kJ/m².

### Example 3

A roll of a meshed knitted fabric band made of fibreglass was submerged in 201 g of the mixture of the example 1. Subsequently, the excess mixture was squeezed out of the band, resulting in 45% band mass increase compared to a non-soaked dressing. The soaked band formed in this way was protected by a sealing package.

Rectangular templates for mechanical tests with dimensions of 25 mm x 80 mm and approx. 4 mm in thickness, consisting of 3 overlapping layers of soaked fibreglass textile, were cut out of the formed soaked band. Upon irradiating for 3 minutes with UV radiation of the wavelength of 405 nm, followed by moistening with water, dust-dry and rigid templates were produced. Mechanical tests of flexural strength were performed (according to the standard PN-EN ISO 178) along with resilience using Charpy's method (according to the standard PN - EN ISO 179-1). The templates were characterised by flexural strength of 1.8 MPa and resilience of 8.2 kJ/m².

### Example 4

The procedure of Example 3 was performed, however, using the mixture of the example 2.

Subsequently, the excess mixture was squeezed out of the band, resulting in 500-600% band mass increase compared to a non-soaked dressing. The soaked band formed in this way was protected by a sealing package

The templates obtained in the same form and tested in the same way as in the example 3, were characterised by flexural strength of 1.41 MPa and resilience of 7.07 kJ/m².

### Example 5

A meshed, knitted fabric textile sleeve made of polyester-elastomer yarn like in example 1 was submerged in 202 g of a mixture comprising:
(a) 100 g of bisphenol A ethoxylate dimethacrylate (CAS no.: 41637-38-1)
(b) 1g of diphenyl(2,4,6-trimethylbenzoyl)phosphine oxide (TPO; CAS no.: 75980-60-8) as a photoinitiator,
(c) 100 g of polyurethane prepolymer partially functionalised with 2-hydroxyethyl methacrylate (HEMA),
(d) 1 g of bis[2-N,Ndimethylamino)ethyl]ether.

Subsequently, the excess mixture was squeezed out of the sleeve, resulting in 500-600% sleeve mass increase compared to a non-soaked dressing. The soaked sleeve formed in this way was protected by a sealing package.

The templates obtained in the same form and tested in the same way as in the example 1, were characterised by flexural strength of 1.55 MPa and resilience of 8.2 kJ/m².

### Example 6

A meshed, knitted fabric textile sleeve made of polyester-elastomer yarn like in example 1 was submerged in 202 g of a mixture comprising
(a) 100 g of bisphenol A ethoxylate dimethacrylate (CAS no.: 41637-38-1)
(b) 1g diphenyl(2,4,6-trimethylbenzoyl)phosphine oxide (TPO; CAS no.: 75980-60-8) as a photoinitiator,
(c) 100 g of polyurethane prepolymer partially functionalised with 2-hydroxyethyl methacrylate (HEMA),
(d) 1 g of 2,2-dimorpholinodiethylether (DMDEE; CAS no.: 6425-39-4)

Subsequently, the excess mixture was squeezed out of the sleeve, resulting in 500-600% sleeve mass increase compared to a non-soaked dressing. The soaked sleeve formed in this way was protected by a sealing package.

The templates obtained in the same form and tested in the same way as in the example 1, were characterised by flexural strength of 1.6 MPa and resilience of 7.8 kJ/m².

### Example 7

A sub-cast sleeve i.e. a substrate element of the immobilisation dressing, made of polyester-elastomer yarn [of 300DEN F96 x 2 polyester thread of raw textured white heavy strain polyester and a polyether-polyurethane copolymer (PA06 78/24/2 DTX Polyurethane (Spandex) 22 DTX Double strain textured polyamide)], was put on the segment of the patient's limb intended for immobilisation. Subsequently, the meshed sleeve of the example 1 soaked in a hybrid mixture comprising components (i)-(iii) was slid onto the substrate element. The limb was placed under a UV lamp with a wavelength of 405 nm in order to cure the outer structure of the dressing. Next, the limb along with the preliminarily surface-cured dressing structure was submerged in water in order to provide complete curing. A waterproof immobilisation dressing was produced in form of a rigid meshed structure having a shape precisely reflecting the surface of the protected segment of the patient's limb.

The hybrid mixture, when exposed to water/moisture from the air (with no UV light) started gelling after 15 minutes, but this process was very slow, and as a result the dressing retained its plasticity until the curing by UV lamp was effected. However, once the limb with the dressing was placed under the UV lamp the preliminary surface curing of the dressing, ensuring sufficient immobilization of the limb took 5 minutes (forearm dressing in size M required 3 minutes).

### Example 8

The immobilisation of the patient's limb was performed in an analogical manner as in the example 4, using the sleeve as described in the example 1, with the proviso, that instead of bis[2-N,Ndimethylamino)ethyl]ether, the mixture used to soak the sleeve comprised dibutyltin dilaurate (IV) (DBTDL) catalysing the reaction of the -NCO and -OH groups, and after the preliminary curing of the dressing outer surface under a UV lamp, instead of submerging the limb with the dressing in water, it was left to dry without supplying additional moisture. A waterproof immobilisation dressing was produced in form of a rigid meshed structure with a shape precisely reflecting the surface of the protected segment of the patient's limb.

The hybrid mixture, when exposed to air (with no UV light and no water) started gelling after 15 minutes, but this process was very slow, and as a result the dressing retained its plasticity until the curing by UV lamp was effected.

However, once the limb with the dressing was placed under the UV lamp the preliminary surface curing of the dressing, ensuring sufficient immobilization of the limb took 5 minutes.

## Claims

1. A curable immobilisation cast dressing comprising a substrate element in form of a flexible sleeve made of knitted fabric with a meshed weave, and a curable element in form of a flexible sleeve made of knitted fabric with a meshed weave, soaked in a curable material, intended to be slid onto the substrate element, both elements being made of synthetic yarn with hydrophobic properties, **characterised in that** the curable material is a hybrid mixture comprising:
(a) at least one compound undergoing polymerisation and/or crosslinking upon exposure to light, preferably with a wavelength of 405 nm, forming at least 50% by weight of the hybrid mixture;
(b) at least one photoinitiator;
(c) at least one compound undergoing polymerisation and/or crosslinking upon exposure to water;
(d) at least one catalyst of the reaction of polymerisation and/or crosslinking of the compound (c) upon exposure to water.

2. The dressing according to claim 1, **characterised in that** the compound (a) undergoing polymerisation and/or crosslinking upon exposure to light at the same time is the compound (c) undergoing polymerisation and/or crosslinking upon exposure to water.

3. The dressing according to claim 1, **characterised in that** the compound (c) undergoing polymerisation and/or crosslinking upon exposure to water is used in an amount of at least 20% by weight of the hybrid mixture.

4. The dressing according to one of the claims 1-3, **characterised in that** at least one compound (c) undergoing polymerisation and/or crosslinking upon exposure to water comprises isocyanate groups.

5. The dressing according to claim 4, **characterised in that** a tertiary amine is used as the catalyst (d), preferably bis[2-N,Ndimethylamino)ethyl]ether or 2,2-dimorpholinodiethylether.

6. The dressing according to one of the claims 1-5, **characterised in that** the curable element along its longitudinal axis has a cutting line defined by the thinner weave of the knitted fabric.

7. The dressing according to one of the claims 1-6, **characterised in that** the substrate element has a lateral branch in form of a sleeve with a smaller diameter, constituting a thumb guard, while the curable element has an opening for the thumb.

8. The dressing according to one of the claims 1-7, **characterised in that** it has a colour marking defining its size, preferably in form of a colour thread finish.

9. The dressing according to claim 8, **characterised in that** the colour marking additionally indicates the location of the opening for the thumb.

## Patentansprüche

1. Ein härtbarer, immobilisierender Gipsverband, welches Folgendes umfasst: ein Substratelement in Form einer biegsamen Hülle aus Maschenware mit Maschenbindung und ein härtbares Element in Form einer biegsamen Hülle aus Maschenware mit Maschenbindung, die mit einem härtbaren Material getränkt ist und dazu bestimmt ist, auf das Substratelement geschoben zu werden, wobei beide Elemente aus synthetischem Garn mit hydrophoben Eigenschaften hergestellt sind, **dadurch gekennzeichnet, dass** das härtbare Material ein Hybridgemisch ist, welches Folgendes umfasst:
(a) mindestens eine Verbindung, die bei Bestrahlung mit Licht, vorzugsweise mit einer Wellenlänge von 405 nm, polymerisiert und/oder vernetzt wird und mindestens 50 Gew.-% der Hybridmischung bildet;
(b) mindestens einen Photoinitiator;
(c) mindestens eine Verbindung, die unter Einwirkung von Wasser polymerisiert und/oder vernetzt;
(d) mindestens einen Katalysator für die Polymerisations- und/oder Vernetzungsreaktion der Verbindung (c) bei der Einwirkung von Wasser.

2. Der Verband nach Anspruch 1, **dadurch gekennzeichnet, dass** die Verbindung (a), die unter Lichteinwirkung polymerisiert und/oder vernetzt, gleichzeitig die Verbindung (c) ist, die unter Wassereinwirkung polymerisiert und/oder vernetzt.

3. Der Verband nach Anspruch 1, **dadurch gekennzeichnet, dass** die Verbindung (c), die unter Einwirkung von Wasser polymerisiert und/oder vernetzt, in einer Menge von mindestens 20 Gew.-% der Hybridmischung verwendet wird.

4. Der Verband nach einem der Ansprüche 1 bis 3, **dadurch gekennzeichnet, dass** mindestens eine Verbindung (c), die unter Einwirkung von Wasser polymerisiert und/oder vernetzt, Isocyanatgruppen enthält.

5. Der Verband nach Anspruch 4, **dadurch gekennzeichnet, dass** ein tertiäres Amin als Katalysator (d) verwendet wird, vorzugsweise Bis[2-N,Ndimethylamino)ethyl]ether oder 2,2-Dimorpholinodiethylether.

6. Der Verband nach einem der Ansprüche 1-5, **dadurch gekennzeichnet, dass** das härtbare Element entlang seiner Längsachse eine Schnittlinie aufweist, die durch die dünnere Bindung der Maschenware definiert ist.

7. Der Verband nach einem der Ansprüche 1-6, **dadurch gekennzeichnet, dass** das Substratelement eine seitliche Abzweigung in Form einer Hülse mit kleinerem Durchmesser aufweist, die einen Daumenschutz bildet, während das härtbare Element eine Öffnung für den Daumen aufweist.

8. Der Verband nach einem der Ansprüche 1-7, **dadurch gekennzeichnet, dass** er eine Farbmarkierung aufweist, die seine Größe definiert, vorzugsweise in Form eines farbigen Fadenabschlusses.

9. Der Verband nach Anspruch 8, **dadurch gekennzeichnet, dass** die Farbmarkierung zusätzlich die Lage der Öffnung für den Daumen angibt.

## Revendications

1. Pansement coulé d'immobilisation durcissable comprenant un élément de substrat sous la forme d'un manchon flexible en tissu tricoté à mailles, et un élément durcissable sous la forme d'un manchon flexible en tissu tricoté à mailles, imbibé d'un matériau durcissable, destiné à être glissé sur l'élément de substrat, les deux éléments étant constitués d'un fil synthétique aux propriétés hydrophobes, **caractérisé en ce que** le matériau durcissable est un mélange hybride comprenant :
(a) au moins un composé subissant une polymérisation et/ou une réticulation lors de l'exposition à la lumière, de préférence à une longueur d'onde de 405 nm, formant au moins 50% en poids du mélange hybride ;
(b) au moins un photo-initiateur ;
(c) au moins un composé subissant une polymérisation et/ou une réticulation lors de l'exposition à l'eau ;
(d) au moins un catalyseur de la réaction de polymérisation et/ou de réticulation du composé (c) lors de l'exposition à l'eau.

2. Pansement selon la revendication 1, **caractérisé en ce que** le composé (a) subissant une polymérisation et/ou une réticulation lors de l'exposition à la lumière est en même temps le composé (c) subissant une polymérisation et/ou une réticulation lors de l'exposition à l'eau.

3. Pansement selon la revendication 1, **caractérisé en ce que** le composé (c) subissant une polymérisation et/ou une réticulation lors de l'exposition à l'eau est utilisé dans une proportion d'au moins 20 % en poids du mélange hybride.

4. Pansement selon l'une des revendications 1 à 3, **caractérisé en ce qu'**au moins un composé (c) subissant une polymérisation et/ou une réticulation lors de l'exposition à l'eau comprend des groupes isocyanates.

5. Pansement selon la revendication 4, **caractérisé en ce qu'**on utilise comme catalyseur (d) une amine tertiaire, de préférence le bis[2-N,Ndiméthylamino)éthyl]éther ou le 2,2-dimorpholinodiéthyléther.

6. Pansement selon l'une des revendications 1 à 5, **caractérisé en ce que** l'élément durcissable présente, le long de son axe longitudinal, une ligne de coupe définie par l'armure la plus fine du tissu tricoté.

7. Pansement selon l'une des revendications 1 à 6, **caractérisé en ce que** l'élément de substrat présente une branche latérale sous forme de manchon de plus petit diamètre, constituant un protège-pouce, tandis que l'élément durcissable présente une ouverture pour le pouce.

8. Pansement selon l'une des revendications 1 à 7, **caractérisé par** un marquage de couleur définissant sa taille, de préférence sous la forme d'un fil de couleur.

9. Pansement selon la revendication 8, **caractérisé en ce que** le marquage de couleur indique en outre l'emplacement de l'ouverture pour le pouce.
